# EUROPEAN PATENT SPECIFICATION

(11) **EP 4 282 322 B1**
(45) Date of publication and mention of the grant of the patent: **11.11.2026**
(21) Application number: 22763491.2
(22) Date of filing: 11.02.2022
(51) Int. Cl.: A61B 5/00, A61B 5/11

(54) **METHOD FOR PROVIDING INFORMATION ABOUT SLEEP QUALITY, AND ELECTRONIC DEVICE SUPPORTING SAME**
VERFAHREN ZUR BEREITSTELLUNG VON INFORMATIONEN ÜBER DIE SCHLAFQUALITÄT UND ELEKTRONISCHE VORRICHTUNG ZUR UNTERSTÜTZUNG DAVON
PROCÉDÉ DE FOURNITURE D'INFORMATIONS CONCERNANT LA QUALITÉ DU SOMMEIL, ET DISPOSITIF ÉLECTRONIQUE LE PRENANT EN CHARGE

(30) Priority: 05.03.2021 KR 20210029201
(43) Date of publication of application: 29.11.2023
(73) Proprietor: Samsung Electronics Co., Ltd., Suwon-si, Gyeonggi-do 16677 (KR)
(72) Inventor: PARK, Yumi, Suwon-si Gyeonggi-do 16677 (KR); HAN, Jeongyup, Suwon-si Gyeonggi-do 16677 (KR)
(74) Representative: Appleyard Lees IP LLP
(86) International application number: PCT/KR2022/002107
(87) International publication number: WO 2022/186507

(56) References cited:
- WO-A1-2015/061579
- JP-A- 2019 531 801
- JP-B2- 6 698 521
- KR-A- 20170 074 364
- KR-A- 20180 126 925
- KR-A- 20200 094 344
- US-A1- 2016 151 603
- US-A1- 2018 078 197
- US-A1- 2019 099 582
- US-A1- 2019 336 077

## Description

### Technical Field

The disclosure relates to a method for providing information about sleep quality and an electronic device supporting the same.

### Background Art

Recently, interest in health and interest in exercise for maintaining health are increasing. Accordingly, electronic devices have been developed to measure and utilize various biometric signal of the human body. The electronic devices may provide various services for health management by identifying a bio-state through measurement of various biometric signals.

A person sleeps for about one-third of his or her life, and if the person fails to sleep, memory of the person may decrease, emotions may become unstable, and hallucinations may occur as a period of insomnia is prolonged. Therefore, since sleep plays an important role in a healthy life, sleep management is desired. US 2018/0078197 A1 describes a sleep scoring device. US 2016/0151603 A1 describes a processing system including methods for promoting sleep. US 2019/0099582 A1 describes sleep performance systems and methods of use thereof. WO 2015/061579 A1 describes methods of using a biometric device. US 2019/0336077 A1 describes determining a health status that includes using an implantable medical device configured for subcutaneous implantation and comprising at least one optical sensor.

### Detailed Description of the Invention

### Technical Challenges

Sleep quality may be evaluated using an index (e.g., Pittsburgh sleep quality index (PSQI)) calculated through a questionnaire for a user. In addition, the sleep quality may be determined based on sleep information (e.g., movement information during sleep, sleep time, and sleep stage) measured through a wearable device.

Related art methods of evaluating sleep quality using an index calculated through the questionnaire for the user have issues such as the sleep quality being determined by the user's subjective input.

### Technical Solution

The invention is set out in the appended claims. Provided are a method for providing information about sleep quality such that sleep quality may be evaluated based on various information related to sleep and obtainable through a wearable device and/or a user input, and an electronic device supporting the same.

Additional aspects will be set forth in part in the description which follows and, in part, will be apparent from the description, or may be learned by practice of the presented embodiments.

### Effects of the Invention

### Brief Description of Drawings

The above and other aspects, features, and advantages of certain embodiments of the present disclosure will be more apparent from the following description taken in conjunction with the accompanying drawings, in which:
FIG. 1 is a block diagram illustrating an electronic device within a network environment according to according to various embodiments;
FIG. 2 is a diagram illustrating a system including an external electronic device and the electronic device according to various embodiments;
FIG. 3 is a block diagram illustrating an electronic device according to various embodiments;
FIG. 4 is a flowchart illustrating a method of providing information about sleep quality according to various embodiments;
FIG. 5 is a flowchart illustrating a method of correcting a sleep score based on a sleep score distribution obtained during a designated period according to various embodiments;
FIG. 6 is a flowchart illustrating a method of determining a user's sleep quality based on obtained sleep-related information and an amount of beverage intake according to various embodiments;
FIG. 7 is a flowchart illustrating a method of determining a sleep grade of a user in a group according to various embodiments;
FIG. 8 is a diagram illustrating a method of determining a sleep grade of a user in a group according to various embodiments; and
FIG. 9 is a diagram illustrating a method of providing information about sleep quality according to various embodiments.

### Mode for Carrying Out the Invention

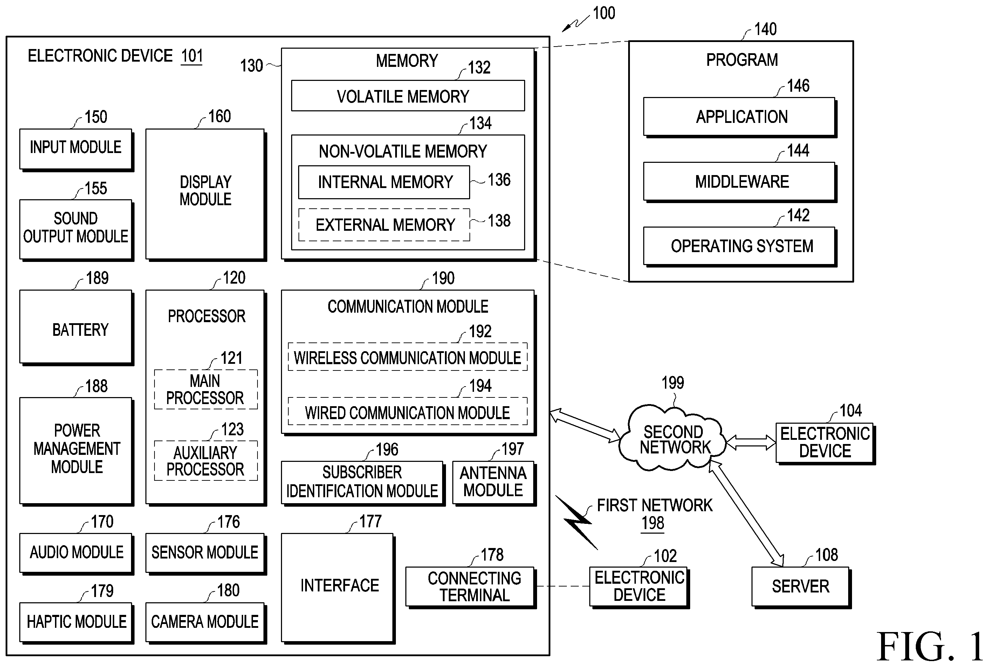

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control, for example, at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active (e.g., executing an application) state. According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence model is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or an external electronic device (e.g., an electronic device 102 (e.g., a speaker or a headphone)) directly or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the AP) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or loT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B", "at least one of A and B", "at least one of A or B", "A, B, or C", "at least one of A, B, and C", and "at least one of A, B, or C", may include any one of, or all possible combinations of the items enumerated together in a corresponding one of the phrases. As used herein, such terms as "1st" and "2nd", or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic", "logic block", "part", or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

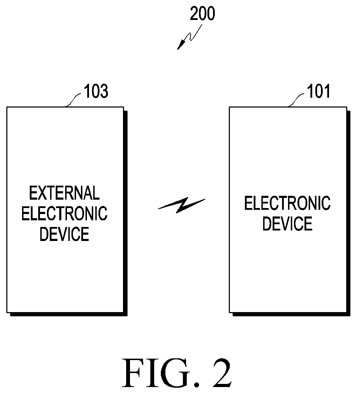

FIG. 2 is a diagram illustrating a system 200 including an external electronic device 103 and the electronic device 101 according to various embodiments.

Referring to FIG. 2, in an embodiment, the external electronic device 103 may be an electronic device capable of being communicatively connected to the electronic device 101. For example, the external electronic device 103 may be the electronic device 102 or the electronic device 104 of FIG. 1.

In an embodiment, the external electronic device 103 may be a wearable device that may be worn on a user (e.g., the user's body). In an embodiment, the external electronic device 103 may obtain sleep-related information (hereinafter referred to as "sleep-related information") before the user goes to sleep (before sleeping) and/or while the user is in a sleep state.

In an embodiment, the external electronic device 103 may obtain sleep-related information including at least one of an exercise amount, stress, or saturation pulse oxygen (SpO2) of the user through a sensor module.

In an embodiment, the external electronic device 103 may obtain information about the exercise amount (e.g., calorie consumption, a type of the exercise, and/or a time of the exercise) of the user by obtaining user motion information using an acceleration sensor. In an embodiment, the external electronic device 103 may obtain information about the amount of exercise in each of a plurality of designated time ranges during a day. For example, the external electronic device 103 may obtain, based on the user's motion information obtained for a period of time from a time when the user ends sleep to a designated time (e.g., 6:00 PM), information about a first exercise amount, and obtain, based on the user's motion information obtained for a period of time from the designated time (e.g., 6:00 PM) to a time when the user starts to sleep, information about a second exercise amount.

In an embodiment, the external electronic device 103 may obtain, using a heart rate sensor, information about a heart rate (HR), heart rate variability, and/or a pulse wave analysis (PWA) feature. For example, the external electronic device 103 may obtain information about the heart rate, the heart rate variability, and/or the PWA feature, based on a signal obtained through a photoplethysmogram (PPG) sensor (or an electrocardiography (ECG) sensor). The external electronic device 103 may obtain, based on the obtained information about the heart rate, the heart rate variability, and/or the PWA feature, information about the stress (e.g., a stress index). In an embodiment, the information about the stress may be information about average daily stress of the user. However, the information about the stress is not limited to the above example. For example, the information about the stress may include information about a change in stress before and after the user goes to sleep.

In an embodiment, the external electronic device 103 may obtain a PPG signal using a PPG sensor (e.g., the PPG sensor using red light and infrared light). The external electronic device 103 may obtain information about saturation pulse oxygen based on the obtained PPG signal. For example, the external electronic device 103 may obtain, based on the PPG signal obtained while the user is in a sleep state, information about saturation pulse oxygen. The external electronic device 103 may obtain information about the number of times by which a numerical value of the saturation pulse oxygen decreases by greater than or equal to a designated value (e.g., about 4%) within a designated time period (about 10 minutes) while the user is in a sleep state. For example, if the numerical value of the saturation pulse oxygen decreases from 98% to 94% within about 10 minutes while the user is in the sleep state, the external electronic device 103 may determine that the number of times by which the numerical value of the saturation pulse oxygen decreases by greater than or equal to about 4% is one time.

In an embodiment, the external electronic device 103 may further obtain, through the sensor module, various sleep-related information in addition to at least one of the exercise amount, the stress, and saturation pulse oxygen of the user. For example, the external electronic device 103 may obtain at least one of a time of a third sleep stage in a first sleep cycle, a ratio of the third sleep stage to a total sleep time, a number of sleep cycles in the total sleep time, the total sleep time, a time of a first sleep cycle, an awake time in a first sleep stage, a time of a wake after sleep onset (WASO) in the first sleep stage, a ratio of the time of the WASO to the total sleep time, an awake time in the first sleep cycle, and a ratio of the awake time to the total sleep time. In an embodiment, the first sleep cycle may be a sleep cycle identified first after identifying the user's sleep start. In an embodiment, the third sleep stage may be a sleep stage representing deep non-rapid-eye-movement (REM) sleep among four sleep stages (e.g., a first sleep stage representing awake during sleep, a second sleep stage representing light non-rapid-eye-movement (non-REM) sleep, the third sleep stage representing deep non-REM sleep, and a fourth sleep stage representing rapid-eye-movement (REM) sleep.

In an embodiment, the external electronic device 103 may transmit the obtained sleep-related information to the electronic device 101 through the communication module (e.g., a short-range communication module). In an embodiment, the external electronic device 103 may transmit, to a server (e.g., a server providing services related to a health application) (e.g., the server 108) through the communication module, the obtained sleep-related information directly or via the electronic device 101.

In an embodiment, the electronic device 101 may obtain, from the external electronic device 103 through the communication module 310, the sleep-related information. In addition or alternatively, the electronic device 101 may obtain, through the sensor module 176 comprised in the electronic device 101 of FIG. 1, at least part of the information included in the aforementioned sleep-related information.

In an embodiment, the electronic device 101 may obtain sleep-related information from the external electronic device 103 through the communication module 310. However, the electronic device 101 is not limited thereto. For example, the electronic device 101, through the sensor module 176 included in the electronic device 101 of FIG. 1, may obtain at least part of the information included in the aforementioned sleep-related information. In an embodiment, the electronic device 101 may determine the user's sleep quality based on the obtained sleep-related information. For example, the electronic device 101 may determine (e.g., calculate) a sleep score by scoring each of values of a plurality of factors included in the obtained sleep-related information. For another example, the electronic device 101 may correct, based on sleep score distribution which has been obtained during a designated period (e.g., sleep scores which has been obtained every day for a month or a week), the sleep score (e.g., a currently (today) determined sleep score).

In an embodiment, the electronic device 101 may determine the user's sleep quality based on the obtained sleep-related information and an amount of beverage intake. For example, the electronic device 101 may obtain, based on the user input, information about the amount of the intake of the beverage (e.g., a water, a coffee, and/or an alcoholic beverage) consumed by the user. The electronic device 101 may determine, based on the obtained sleep-related information and beverage intake, the user's sleep score.

In an embodiment, the electronic device 101 may obtain user information. For example, the electronic device 101 may obtain information about the user's gender, age, and/or height/weight as the user information. Based on the obtained user information, the electronic device 101 may obtain information about a sleep score distribution of a group (e.g., other users) having user information that is the same as or similar to the obtained user information. If the information about the sleep score distribution of the group is obtained, the electronic device 101 may determine a sleep grade of the user in the group by comparing the determined sleep score of the user with the sleep score distribution of the group.

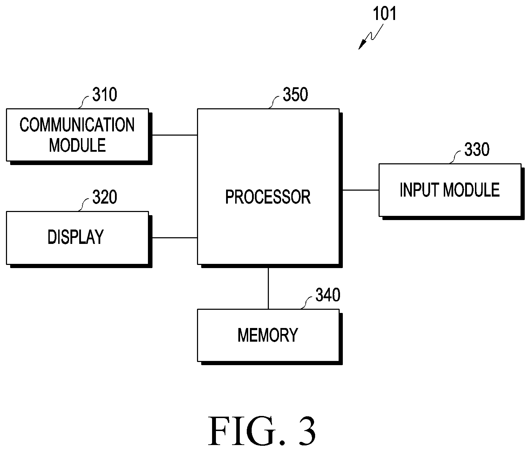

Hereinafter, a method for providing information about the sleep quality will be described in detail with reference of FIGS. 3 to 9.

FIG. 3 is a block diagram of the electronic device 101 according to various embodiments.

Referring to FIG. 3, in an embodiment, an electronic device 101 may include a communication module 310, a display 320, an input module 330, a memory 340, and/or a processor 350. The processor 350 may include one or more processors, and may be referred to herein as "a processor," "at least one processor," "one or more processors," etc.

In an embodiment, a communication module 310 (also referred to as "communication circuitry") may be included in the communication module 190 of FIG.1. For example, the communication module 310 may include a short-range communication module.

In an embodiment, the communication module 310 may receive sleep-related information from the external electronic device 103 communicatively connected to the electronic device 101. For example, the communication module 310 may obtain, from the external electronic device 103, sleep-related information including at least one of an exercise amount, stress, and saturation pulse oxygen of the user. For another example, the communication module 310 may obtain, from the external electronic device 103, sleep-related information including at least one of the exercise amount, the stress, or the saturation pulse oxygen of the user, a time of a third sleep stage in a first sleep cycle, a ratio of the third sleep stage to a total sleep time, a number of sleep cycles in the total sleep time, the total sleep time, a time of a first sleep cycle, an awake time in a first sleep stage, a time of a WASO in the first sleep stage, a ratio of the time of the WASO to the total sleep time, an awake time in the first sleep cycle, and a ratio of the awake time to the total sleep time. Hereinafter, each information included in the sleep-related information may be also referred to "sleep factor value".

In an embodiment, the communication module 310 may receive information about sleep score distribution of a group (e.g., other users) having user information 101 from a server (e.g., a server providing a service related to a health application) (e.g., a server 108) (hereinafter referred to as a "server") communicatively connected to the electronic device 101.

In an embodiment, if the sleep score of the user of the electronic device 101 is determined, the communication module 310 may transmit the determined sleep score to the server 108. For example, if the sleep score of the user of the electronic device 101 is determined, the communication module 310 may transmit the determined sleep score to server in which an account of the user of the electronic device 101is registered and which stores the user information (e.g., the sex, age, and/or height/weight).

In an embodiment, a display 320 may be included in display module 160 of FIG. 1.

In an embodiment, the display 320 may display various information related to the sleep quality. For example, the display 320 may display information about at least one of the determined sleep score (e.g., currently (today) determined sleep score), an average of sleep scores determined during a designated period, an average sleep score of the group having user information the same as or similar to the user information of the user of the electronic device 101, or a value of a sleep factor associated with a decrease in sleep score. However, the information displayed on the display 320 is not limited to the above example.

In an embodiment, an input module 330 may be included in input module 150 of FIG. 1.

In an embodiment, the input module 330 may receive the user information (e.g., the user's gender, age, and/or height/weight) from the user. In an embodiment, the input module 330 may receive information about an amount of beverage intake from the user. However, the information received by the input module 330 from the user is not limited to the above example.

In an embodiment, a memory 340 may be included in memory 130 of FIG. 1.

In an embodiment, the memory 340 may store various information obtained to perform an operation for providing information about the user's sleep quality.

In an embodiment, a processor 350 may be included in the processor 120 of FIG. 1. The processor 350 may include one or more processors to perform an operation of providing information about the user's sleep quality.

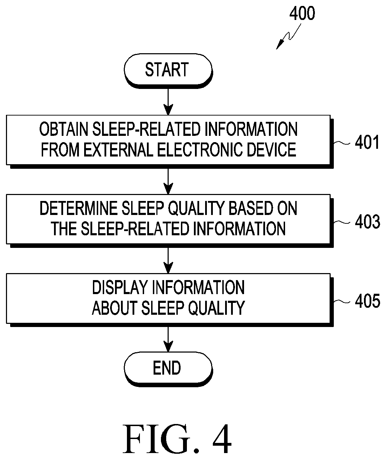

Hereinafter, a method for providing, by the processor 350, information about the user's sleep quality will be described in detail with reference to FIGS. 4 to 9.

In FIG. 3, the electronic device 101 is illustrated as including the communication module 310, the display 320, the input module 330, the memory 340, and the processor 350, but the electronic device 101 not limited thereto. In an embodiment, the electronic device 101 may further include at least one of the components shown in FIG. 1 (e.g., the sensor module 176) or may not include some of the components shown in FIG. 3. In an eFmbodiment, the electronic device 101 may further include a sensor module at least partially the same as a sensor module included in the external electronic device 103 described above through FIG. 2 to obtain sleep-related information. For example, the electronic device 101 may further include an acceleration sensor, a heart rate sensor, and/or a PPG sensor.

The electronic device 101 according to various embodiments of the present disclosure may include a communication circuitry (e.g., the communication module 310), a display 320, and at least one processor 350 functionally connected to the communication circuitry (e.g., communication module 310) and the display 320. The at least one processor 350 may obtain, from an external electronic device through the communication circuitry, sleep-related information including at least one of an exercise amount, a stress, and a saturation pulse oxygen of a user, based on the sleep-related information, determine the sleep quality of the user, and display information about the determined sleep quality of the user through the display 320.

In various embodiments, the sleep-related information may further include at least one of a time of a third sleep stage in a first sleep cycle, a ratio of the third sleep stage to a total sleep time, a number of sleep cycles in the total sleep time, the total sleep time, a time of a first sleep cycle, an awake time in a first sleep stage, a time of a WASO in the first sleep stage, a ratio of the time of the WASO to the total sleep time, an awake time in the first sleep cycle, and a ratio of the awake time to the total sleep time.

In various embodiments, the at least one processor 350 may be configured to calculate a sleep score of the user, by performing scoring for each of information included in the sleep-related information.

In various embodiments, the at least one processor 350 may be configured to add a value to a score corresponding to an amount of exercise obtained before a designated time and subtract a value from a score corresponding to an amount of exercise obtained after the designated time.

In various embodiments, the at least one processor 350 may be configured to assign a lower score as the stress increases.

In various embodiments, the at least one processor 350 may be configured to assign a lower score as the stress of the user increases and assign a lower score based on a number of times being higher, the number of times being a number of times by which the stress of the user decreases being greater than or equal to a designated value within a designated time during sleep.

In various embodiments, the at least one processor 350 may be configured to, based on an average and standard deviation of sleep scores obtained during a designated period of time (e.g., sleep quality information about the user), calculate a designated range related to a sleep score, if the calculated sleep score is less than a lower limit value of the designated range, correct the calculated sleep score to the lower limit value, and if the calculated sleep score is less than an upper limit value of the designated range, correct the calculated sleep score to the upper limit value.

In various embodiments, the at least one processor 350 may be configured to, based on a user input, obtain information about an amount of beverage intake of a user, and based on the amount of the beverage intake, the sleep-related information and the sleep quality information, and determine the sleep quality of the user.

In various embodiments, the at least one processor 350 may be configured to obtain a sleep score distribution of a group corresponding to the user information, and based on the user information and the sleep score distribution of the group, determine a sleep grade of the user in the group.

In various embodiments, the user information may include a gender, an age, and/or a height/weight of the user.

FIG. 4 is a flowchart 400 illustrating a method of providing information about sleep quality according to various embodiments.

Referring to FIG. 4, in operation 401, in an embodiment, the processor 350 may obtain sleep-related information from the external electronic device 103 through the communication module 310.

In an embodiment, the processor 350 may obtain sleep-related information including at least one of the exercise amount (the amount of the exercise), the stress, and the saturation pulse oxygen of the user from the external electronic device 103 through the communication module 310.

In an embodiment, the exercise amount of the user may be obtained by the external electronic device 103, based on user motion information obtained using an acceleration sensor of the external electronic device 103. However, a sensor for obtaining the exercise amount of the user is not limited to the acceleration sensor. In an embodiment, the exercise amount of the user may include a first exercise amount and a second exercise amount, the first exercise amount being obtained based on the user's motion information which has been obtained for a period of time from a time when the user ends sleep to a designated time (e.g., 6:00 PM), the second exercise amount being obtained based on the user's motion information which has been obtained based on the user's motion information obtained for a period of time from the designated time (e.g., 6:00 PM) to a time when the user starts to sleep. In an embodiment, the exercise amount of the user may include the user's calorie consumption. Furthermore, the amount of the exercise of the user may include a type of the exercise and/or a time of the exercise.

In an embodiment, the information about the stress of the user may indicate average daily stress of the user. In an embodiment, the external electronic device 103 may obtain information about a heart rate, a heart rate variability, and/or a PWA feature by using a heart rate sensor. For example, the external electronic device 103 may obtain information about the heart rate, the heart rate variability, and/or the PWA feature, based on a signal obtained through a PPG sensor (or an electrocardiography (ECG) sensor). The external electronic device 103 may obtain, based on the obtained information about the heart rate, the heart rate variability, and/or the PWA feature, information about the stress (e.g., a stress index). The external electronic device 103 may transmit the information about the obtained stress to the electronic device 101. In an embodiment, the information about the stress may include information about a change in stress before and after the user goes to sleep.

In an embodiment, the saturation pulse oxygen information may include information about the number of times by which a numerical value of the saturation pulse oxygen decreases by greater than or equal to a designated value (e.g., about 4%) or more within a designated time period (about 10 minutes) while the user is in a sleep state. For example, the external electronic device 103 may obtain a PPG signal using a PPG sensor (e.g., the PPG sensor using red light and infrared light). The external electronic device 103 may obtain information about saturation pulse oxygen based on the obtained PPG signal. For example, the external electronic device 103 may obtain information about saturation pulse oxygen based on the PPG signal obtained while the user is in a sleep state. The external electronic device 103 may obtain information about the number of times by which the numerical value of the saturation pulse oxygen decreases by greater than or equal to the designated value (e.g., about 4%) within the designated time period (about 10 minutes) while the user is in the sleep state. The external electronic device 103 may transmit information about the obtained saturation pulse oxygen to the electronic device 101.

In an embodiment, the processor 350 may obtain, from the external electronic device 103 through the communication module 310, at least one of a time of a third sleep stage in a first sleep cycle, a ratio of the third sleep stage to a total sleep time, a number of sleep cycles in the total sleep time, the total sleep time, a time of a first sleep cycle, an awake time in a first sleep stage, a time of a WASO in the first sleep stage, a ratio of the time of the WASO to the total sleep time, an awake time in the first sleep cycle, and a ratio of the awake time to the total sleep time, in addition to at least one of the amount of the exercise, the stress, or the saturation pulse oxygen of the user.

However, the sleep-related information obtained by the processor 350 is not limited to the above examples.

In the above examples, the processor 350 is exemplified as obtaining sleep-related information from the external electronic device 103 through the communication module 310, but is not limited thereto. For example, the processor 350 may obtain at least some of the information included in the aforementioned sleep-related information through the sensor module 176 included in the electronic device 101.

In operation 403, in an embodiment, the processor 350 may determine sleep quality based on the sleep-related information.

In an embodiment, the processor 350 may calculate a sleep score by comparing a value of at least one sleep factor included in the sleep-related information with designated ranges (or designated values) which are stored in the memory 340 in a form of a table and which are set for at least one sleep factor. For example, the processor 350 may identify a range to which the value of the at least one sleep factor belongs, among the designated ranges which are set for the at least one sleep factor. The processor 350 may calculate the sleep score by identifying a score corresponding to (e.g., mapped to) the identified range.

In an embodiment, the processor 350 may store in the memory 340, a designated range (or designated value) and a score corresponding to the designated range for each of the at least one sleep factor in the form of the table, as shown in Table 1 below. In Table 1, the +/- values may indicate the values used to increase/decrease the score.

**[Table 1]**

| sleep factor | score corresponding to a designated range (or a designated value) | |
|---|---|---|
| exercise amount (Kcal) | In a case that the exercise amount is obtained before the designated time (6:00 PM) | less than 50 (Kcal) : 0 |
| | | greater than or equal to 50 and less than 100 : +1 |
| | | greater than or equal to 100 and less than 200 : +2 |
| | | greater than or equal to 200 : +3 |
| | In a case that the exercise amount is obtained after the designated time (6:00 PM) | less than 50 : 0 |
| | | greater than or equal to 50 and less than 100 : -1 |
| | | greater than or equal to 100 and less than 200 : -2 |
| | | greater than or equal to 200 : -3 |
| stress (average daily stress per day) | greater than or equal to 70 : -3 | |
| | greater than or equal to 60 and less than 70 : -2 | |
| | greater than or equal to 50 and less than 60: -1 | |
| | greater than or equal to than 0 and less than 50: 0 | |
| saturation pulse oxygen (the number of times by which saturation pulse oxygen is reduced by about 4% or more in about 10 minutes) | 0 time: 0 | |
| | 1 time : -1 | |
| | 2 times : -2 | |
| | 3 times : -3 | |
| | 4 times or more : -5 | |
| time of the 3rd sleep stage in the 1st sleep cycle (deep in first sleep cycle) | 0 minute: 0 | |
| | longer than or equal to 0 minutes and shorter than 5 minutes : +7 | |
| | longer than or equal to 5 minutes and shorter than 30 minutes : +10 | |
| | longer than or equal to 30 minutes and shorter than 50 minutes : +15 | |
| | longer than or equal to 50: +20 | |
| ratio of 3rd sleep stage in total sleep time (deep rate in total sleep) | 0 %: 0 | |
| | greater than 0% and less than 5% : +5 | |
| | greater than or equal to 5% and less than 10% : +10 | |
| | greater than or equal to 10% and less than 15% : +12 | |
| | greater than or equal to 15%: +15 | |
| the number of sleep cycles in total sleep time (sleep cycle) | 0 times : 0 | |
| | 1 time and 2 times : +5 | |
| | 3 times to 6 times : +10 | |
| | 7 times or more : +5 | |
| total sleep time | shorter than 6 hours : +4 | |
| | longer than or equal to 6 hours and shorter than 9 hours : +10 | |
| | 9 longer than or equal to : +4 | |
| first sleep cycle time | 0 minutes : 0 | |
| | longer than 0 minutes and shorter than 60 minutes : +5 | |
| | longer than or equal to 60 minutes and shorter than 120 minutes : +10 | |
| | longer than or equal to120 : +5 | |
| awake time during the first sleep stage (wake in REM) | Less than 6 minutes : +10 | |
| | longer than or equal to 6 minutes and shorter than 16 minutes : +5 | |
| | longer than or equal to 16 minutes: 0 | |
| WASO (wake time after sleep onset) in the first sleep stage (WASO in REM) | 0 times : +8 | |
| | 1 time: +5 | |
| | 2 times or more : +2 | |
| ratio of the WASO to the total sleep time (WASO rate in total sleep) | less than 2 % : +6 | |
| | greater than or equal to 2 % and less than 5 % : +3 | |
| | greater than or equal to 5 % : 0 | |
| awake time in the first sleep cycle(wake in first sleep cycle) | less than 2 minutes : +6 | |
| | longer than or equal to 2 minutes and shorter than 8 minutes : +4 | |
| | longer than or equal to 8 minutes and shorter than 45 minutes : +3 | |
| | 45 minutes or more : +1 | |
| ratio of the awake time to the total sleep time | less than 5 % : +5 | |
| | greater than or equal to 5 % and less than 10 % : +4 | |
| | greater than or equal to 10 % and less than 30 %: +3 | |
| | greater than or equal to 30 % : +1 | |

As shown in Table 1, in an embodiment, the processor 350, in the case that the exercise amount is obtained before a designated time (6:00 PM) (e.g., until 6:00 PM after the end of sleep) with respect to the amount of exercise, may store, in the memory 340, scores (0, +1, +2, and +3) (e.g., scores to be added) respectively corresponding to the ranges (less than 50, greater than or equal to 50 and less than 100, greater than or equal to 100 and less than 200, and greater than or equal to 200) of the exercise amount (e.g., calorie consumption (Kcal)). In the case that the exercise amount is obtained after the designated time (6:00 pm) (e.g., from 6:00 pm to before going to bed) with respect to the amount of exercise, the processor 350 may store, in the memory 340, scores (0, -1, -2, and -3) (e.g., scores to be subtracted) respectively corresponding to the ranges (e.g., the ranges of the calorie consumption (Kcal) (less than 50, greater than or equal to 50 and less than 100, greater than or equal to 100 and less than 200, and greater than or equal to 200)). In an embodiment, reflecting that the amount of exercise obtained before the designated time (6:00 PM) has a good effect on the quality of sleep but the amount of exercise obtained after the designated time has a bad effect on the quality of sleep, the processor 350 may assign the higher the score (e.g., the score to be added) corresponding to the amount of exercise obtained before the designated time (6:00 p.m.) is greater, and may assign the lower the score (e.g., points to be subtracted) corresponding to the amount of exercise obtained after the designated time (6:00 p.m.) is greater.

In an embodiment, with respect to stress, the processor 350 may store, in the memory 340, scores (e.g., -3, -2, -1, and 0) respectively corresponding to average daily stress ranges (e.g., greater than or equal to 70, greater than or equal to 60 and less than 70, greater than or equal to 50 and less than 60, and greater than or equal to 0 and less than 50). In an embodiment, the processor 350 may assign a lower score (e.g., a large subtracted score) as the average daily stress is greater, reflecting that a higher average daily stress has a negative effect on sleep quality.

In an embodiment, with respect to the saturation pulse oxygen, the processor 350 may store, in the memory 340, scores (e.g., 0, -1, -2, -3, and -5) respectively corresponding to the number of times (e.g., 0 times, 1 time, 2 times, 3 times, and 4 times or more) by which the saturation pulse oxygen during sleep is reduced by a designated value (4%) or more within a designated time (within about 10 minutes). In an embodiment, with respect to the saturation pulse oxygen, by reflecting that the higher the number of times by which the saturation pulse oxygen during sleep is reduced by greater than or equal to the designated value (4%) within the designated time (within about 10 minutes) adversely affect the quality of sleep (e.g., sleep apnea may be determined if the number of times by which saturation pulse oxygen during sleep decreases by greater than or equal to a designated value (4%) within the designated time (within about 10 minutes) is greater than or equal to the designated number of times), the processor 350 may assign the lower the score, the higher the number of times by which the saturation pulse oxygen during sleep is reduced by greater than or equal to the designated value (4%) within the designated time (within about 10 minutes).

In an embodiment, as shown in Table 1, considering that the first sleep cycle has a greater influence on sleep quality than other sleep cycles, the processor 350 may set designated ranges and scores corresponding to the designated ranges, for each of the sleep factors related to the first sleep cycle (e.g., a time of the third sleep stage in the first sleep cycle, a time of the first sleep cycle, and a awake time in the first sleep cycle).

In an embodiment, as shown in Table 1, considering that the first and third sleep stages have a greater influence on sleep quality than other sleep stages, the processor 350 may set designated ranges and scores corresponding to the designated ranges, for each of the sleep factors associated with the first and third sleep stages (e.g., a time of the third sleep stage in the first sleep cycle, a ratio of the third sleep stage in the total sleep time, an awake time during the first sleep stage, and an wake time after sleep onset in the first sleep stage).

In an embodiment, as shown in Table 1, considering that the first sleep cycle has a greater influence on sleep quality than other sleep cycles, the processor 350 may set the designated ranges and scores corresponding to the designated ranges, for each of the sleep factors (e.g., a time of the third sleep stage in the first sleep cycle, a time of the first sleep cycle, and an awake time in the first sleep cycle) of the first sleep cycle.

In an embodiment, the processor 350 may identify ranges to which respective values of the plurality of sleep factors belong, among designated ranges set for each of the plurality of sleep factors. The processor 350 may determine (e.g., calculate) a sleep score (e.g., a final sleep score) by summing up scores corresponding to the identified ranges.

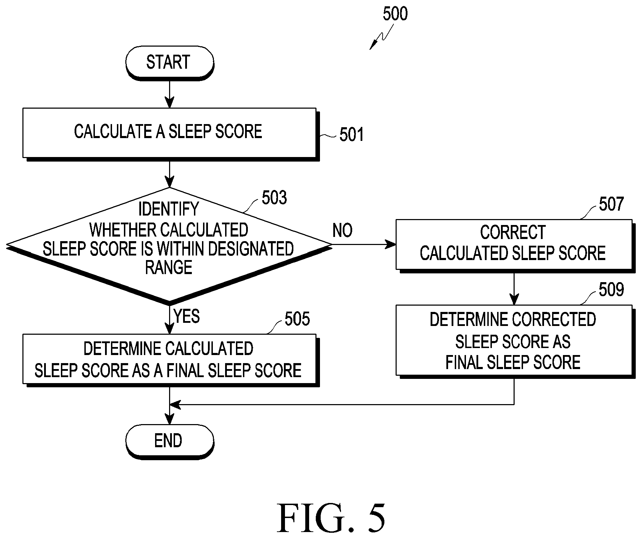

In an embodiment, the processor 350 may correct a sleep score (e.g., currently (today) determined sleep score) based on a distribution of sleep scores obtained during a designated period (e.g., sleep scores obtained daily for a month or a week). A method of correcting the determined sleep score will be described later in detail with reference to FIG. 5.

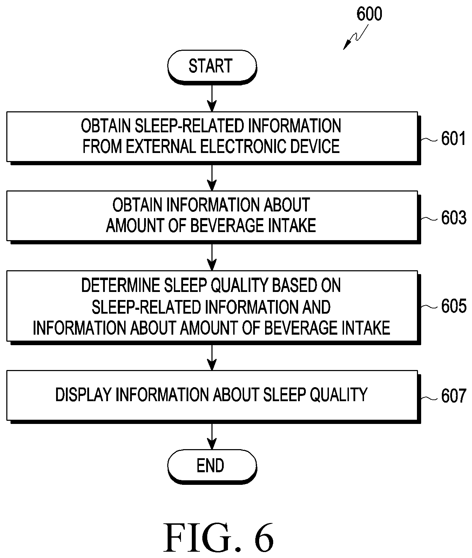

In an embodiment, the processor 350 may determine the user's sleep quality based on the obtained sleep-related information and an amount of beverage intake. For example, the processor 350 may obtain information about an amount of beverage (e.g., a water, a coffee, and/or an alcoholic beverage) intake based on the user input. The processor 350 may determine the user's sleep score based on the obtained sleep-related information and the amount of the beverage intake. A method of determining the user's sleep quality based on the obtained sleep-related information and the amount of the beverage intake will be described in detail later with reference to FIG. 6.

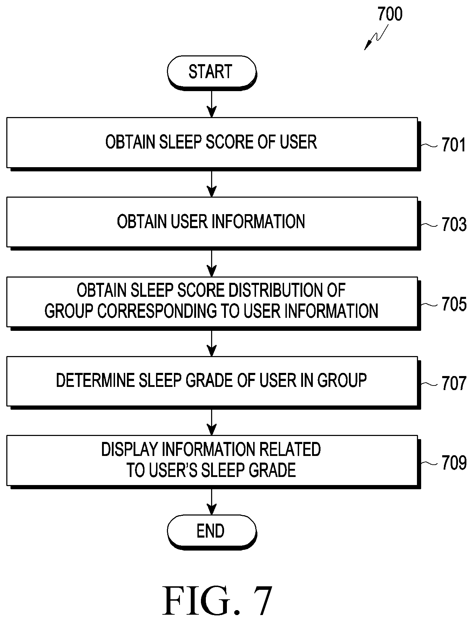

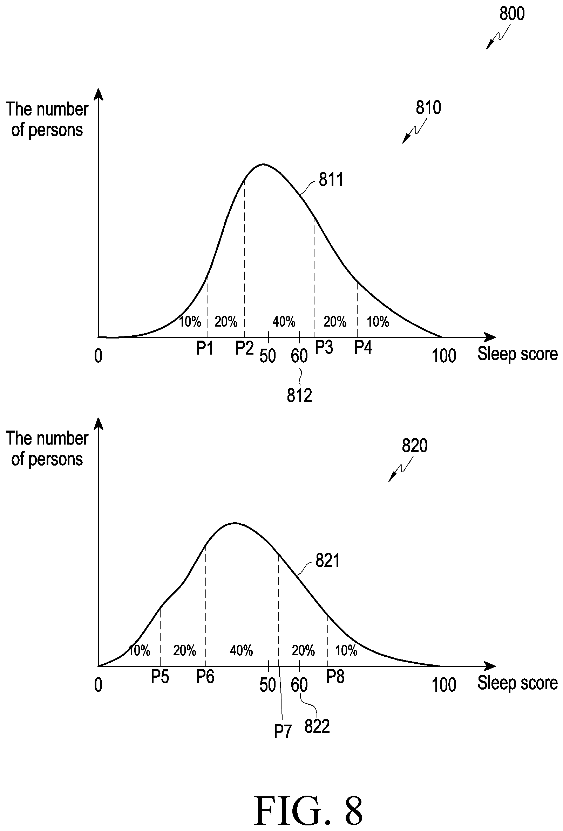

In an embodiment, the processor 350 may obtain, based on the user information, information about a sleep score distribution of a group (e.g., other users) having user information the same as or similar to the obtained user information. If information about the sleep score distribution of the group is obtained, the electronic device 101 may determine a sleep grade of the user in the group by comparing the determined sleep score of the user with the sleep score distribution of the group. A method of determining the sleep grade of the user in the group by comparing the determined sleep score of the user with the sleep score distribution of the group will be described in detail with reference to FIGS. 7 and 8.

In an embodiment, the processor 350 may obtain sleep quality information of the user, the sleep quality information has being been obtained during a designated period of time, obtain user information about the user, and determine a sleep quality of the user based on the sleep-related information, the sleep quality information, and the user information. In an embodiment, the sleep quality information may be obtained by evaluating, using an index (e.g., Pittsburgh sleep quality index (PSQI)) calculated through a questionnaire for a user, sleep quality of the user. In an embodiment, the user information may include a gender, an age, and/or a height/weight of the user.

In operation 405, in an embodiment, the processor 350 may display information about sleep quality through the display 320.

In an embodiment, the processor 350 may display various information related to the sleep quality through the display 320. For example, the processor 350 may display, through the display 320, information about at least one of the sleep score (e.g., the currently (today) determined sleep score), an average of sleep scores determined during a designated period, an average sleep score of the group having user information the same as or similar to user information about the electronic device 101, or a value of a sleep factor related to a decrease (or increase) of the sleep score. However, the information displayed by the processor 350 is not limited to the above example.

FIG. 5 is a flowchart 500 illustrating a method of correcting a sleep score based on a sleep score distribution obtained during a designated period according to various embodiments.

In an embodiment, FIG. 5 may be examples of operations included in or added to operation 403 of FIG. 4.

Referring to FIG. 5, in operation 501, in an embodiment, the processor 350 may calculate a sleep score. For example, as described through operation 403 of FIG. 4, the processor 350 may determine current (today) sleep quality based on the sleep-related information.

In operation 503, in an embodiment, the processor 350 may identify whether the calculated sleep score is within a designated range.

In an embodiment, the processor 350 may obtain a sleep score distribution (e.g. sleep scores obtained every day, within a month or week from now) obtained during a designated period, the sleep score distribution being stored in memory 340 or received from server 108 after stored in server 108. The processor 350 may calculate an average and standard deviation of sleep scores obtained during the designated period. The processor 350 may calculate the designated range, based on the average and the standard deviation of the sleep scores obtained during the designated period. For example, the processor 350 may calculate the designated range based on the average and the standard deviation of the sleep scores obtained during a designated period, using Equations (1) and (2) below. $lower limit value of designated range = mean - \left(n*standard deviation\right)$ (1) $upper limit value of designated range = mean + \left(n*standard deviation\right)$ (2)

[In Equations (1) and (2), the average may represent an average of sleep scores obtained during the designated period, and the standard deviation may represent the standard deviation of the sleep scores obtained during a designated period. In Equations (1) and (2), n may be set to an arbitrary value (e.g., 3).

In an embodiment, if the average of the sleep scores obtained during the designated period is 70, the standard deviation is 8.3, and n is set to 3, the processor 350 may set greater than equal to 45.1 (point) and less than or equal to 94.9 (point) or less as a designated range related to the sleep score.

If the sleep score calculated is within the designated range in operation 503, in an embodiment, in operation 505, the processor 350 may determine the calculated sleep score as a final sleep score.

If the sleep score calculated is not within the designated range in operation 503, in an embodiment, in operation 507, the processor 350 may correct the calculated sleep score.

In an embodiment, if the calculated sleep score (e.g., the sleep score calculated in operation 501) is less than the lower limit value of the designated range, the processor 350 may correct the calculated sleep score to the lower limit value of the designated range. For example, if the calculated sleep score is 30 point, the processor 350 may correct the calculated sleep score of 30 points to 45.1 point as the lower limit value of the designated range.

In an embodiment, if the calculated sleep score (e.g., the sleep score calculated in operation 501) is greater than the upper limit value of the designated range, the processor 350 may correct the calculated sleep score to the upper limit value of the designated range. For example, if the calculated sleep score is 98 point, the processor 350 may correct the calculated sleep score of 98 point to 94.9 point as the upper limit of the designated range.

At operation 509, in an embodiment, the processor 350 may determine the corrected sleep score as a final sleep score.

In an embodiment, a case that the calculated sleep score is not within the designated range may be an exceptional case in the evaluation of the user's sleep quality (or a case that distribution for the user's sleep quality is not ideal). The personalized sleep score may be obtained by correcting, by the processor 350 and based on the sleep score distribution obtained during the designated period, the sleep score.

FIG. 6 is a flowchart 600 illustrating a method of determining a user's sleep quality based on obtained sleep-related information and an amount of beverage intake according to various embodiments.

Referring to FIG. 6, in operation 601, in an embodiment, the processor 350 may obtain sleep-related information from the external electronic device 103 through the communication module 310.

Since operation 601 is at least partially the same as or similar to operation 401, repeated descriptions may be omitted.

In operation 603, in an embodiment, the processor 350 may obtain information about an amount of the beverage intake.

In an embodiment, the processor 350 may obtain, using the input module 330, information about the amount of the beverage intake from the user. For example, the processor 350 may obtain, based on a user input, information about an amount of intake of a water, a coffee, and/or an alcoholic beverage.

In operation 605, in an embodiment, the processor 350 may determine the sleep quality, based on the sleep-related information and information about the amount of the beverage intake.

In an embodiment, the processor 350 may calculate a sleep score based on a value of the sleep factor included in the sleep-related information and the amount of the beverage intake. In an embodiment, the processor 350 may calculate a sleep score, by comparing the value of the sleep factor included in the sleep-related information and the amount of the beverage intake with designated ranges (or designated values), the designated ranges being stored in a form of a table in the memory 340 and being set for at least one sleep factor and beverage. For example, the processor 350 may identify a range to which a value of at least one sleep factor and the amount of the beverage intake belong, among designated ranges set for the at least one sleep factor and the beverage. The processor 350 may calculate the sleep score by identifying a score corresponding to (e.g., mapped to) the identified range.

In an embodiment, since the designated range (or designated value) and the score corresponding to the designated range, set for each of the at least one sleep factor included in the sleep-related information have been described above through Table 1, description thereof will be omitted.

In an embodiment, in addition to Table 1, as shown in Table 2 below, the processor 350 may store, in the memory 340, a designated range (or designated value) set for each beverage in table form and a score corresponding to the designated range. In Table 2, the +/- values may indicate the values used to increase/decrease the score.

**[Table 2]**

| beverage | score corresponding to designated range (or designated value) |
|---|---|
| water | greater than equal to 1 (L(liter)) : +1 |
| | less than 1 (L) : 0 |
| coffee | less than equal to 250 (mL) : 0 |
| | greater than 250 (mL) and less than or equal to 500 (mL) : -1 |
| | greater than 500 (mL) and less than or equal to 750 (mL) : -2 |
| | greater than 750 (mL) : -3 |
| alcoholic beverage | 350 (mL) or less : 0 |
| | greater than 350 (mL) and less than or equal to 700 (mL) : -1 |
| | greater than 700 (mL) and less than or equal to 1050 (mL) : -2 |
| | greater than 1050 (mL) : -3 |

As shown in Table 2, in an embodiment, with respect to water intake, the processor 350 may assign a higher score for an amount of a water intake of greater than equal to 1 (L), compared to an amount of a water intake of less than 1 (L). With respect to coffee intake and alcoholic beverage intake, the processor 350 may assign a lower score as an amount of the coffee intake and an amount of alcoholic beverage intake increase. In an embodiment, the processor 350 may identify ranges to which each of values of a plurality of sleep factors and each of amounts of the beverage intake belongs among the designated ranges set for each of the plurality of sleep factors and each of the amounts of the beverage intake. The processor 350 may determine (e.g., calculate) a sleep score (e.g., a final sleep score) by summing scores corresponding to the identified ranges.

In operation 607, in an embodiment, the processor 350 may display information about sleep quality through the display 320.

Since operation 607 is at least partially the same as or similar to operation 405, repeated descriptions may be omitted.

FIG. 7 is a flowchart 700 illustrating a method of determining a sleep grade of a user in a group according to various embodiments.

FIG. 8 is a diagram 800 for illustrating a method of determining a sleep grade of a user in a group according to various embodiments.

Referring to FIGS. 7 and 8, in operation 701, in an embodiment, the processor 350 may obtain a sleep score of the user. For example, as described with reference to FIGS. 2 to 6, the processor 350 may obtain the sleep score of the user by determining, based on sleep-related information (and beverage intake), the sleep score of the user.

At operation 703, in an embodiment, processor 350 may obtain user information. For example, the processor 350 may obtain information about the user's gender, age, and/or height/weight, based on the user input.

In operation 705, in an embodiment, the processor 350 may obtain a sleep score distribution of a group corresponding to the user information. For example, the processor 350 may obtain, from the server 108 (e.g., the server 108 providing a service related to a health application) (e.g., the server 108) through the communication module, information about the sleep score distribution of the group corresponding to the user information.

In an embodiment, the group corresponding to the user information may be a group including other users having user information the same as or similar to the user information about the user of the electronic device 101. For example, if the user information about the user of the electronic device 101 indicates that the gender is male, the age is 35, and the body mass index (BMI) is about 23.1 (e.g., height/weight is 180 cm/75 kg), the group corresponding to the user information may be a group including other users whose genders are male, whose ages are greater than or equal to 30 and less than 40, and whose body mass indices are greater than or equal to 20 and less than 30.

In operation 707, in an embodiment, the processor 350 may determine a sleep grade of the user in the group, based on the user's sleep score, the user information, and the sleep score distribution of the group.

In an embodiment, the processor 350 may determine the sleep grade of the user in the group by identifying a grade corresponding to the sleep score of the user in the sleep score distribution of the group. For example, graph 810 in FIG. 8 shows a sleep score distribution 811 of a group (e.g., a group including users whose genders are male, whose ages are greater than or equal to 30 years old and less than 40 years old, and whose body mass index are greater than or equal to 20 and less than 30) corresponding to the user information about the user (e.g., whose gender is male, whose age is 35 years old, whose body mass index is about 23.1 (e.g., height/weight are 180 cm/75 kg)) of the electronic device 101. As shown by graph 810, a user with a sleep score greater than equal to 0 and less than P1 may be a bottom 10% (grade 5) within the group, and a user with a sleep score greater than or equal to P1 and less than P2 may be greater than or equal to the bottom 10% and less than 30% (grade 4) within the group, a user with a sleep score greater than or equal to P2 and less than P3 may be greater than or equal to the bottom 30% and less than or equal to top 30% (grade 3) within the group, a user with a sleep score greater than or equal to P3 and less than P4 may be greater than or equal to the top 30% and less than top 10% (grade 2) within the group, and a user with a sleep score greater than or equal to P4 may be greater than or equal to the top 10% (grade 1) within the group. If the sleep score of the user of the electronic device 101 is 60 (point 812), the processor 350 may determine the sleep grade of the user of the electronic device 101 as 3 grade.

For another example, graph 820 in FIG. 8 shows a sleep score distribution 821 of a group (e.g., the group including users whose genders are male, whose ages are greater than or equal to 50 years old and less than 60 years old, and whose body mass indices greater than or equal to 20 and less than 25) corresponding to user information about the user (e.g., whose gender is male, whose age is 55 years old, and whose body mass index is about 22.5 (e.g. height/weight is 170 cm/65 kg)) of the electronic device 101. As shown by graph 820, a user with a sleep score greater than or equal to 0 and less than P5 may be the bottom 10% (grade 5) within the group, a user with a sleep score greater than or equal to P5 and less than P6 may be greater than or equal to the bottom 10% and less than bottom 30% (grade 4) within the group, a user with a sleep score greater than or equal to P6 and less than P7 may be greater than or equal to the bottom 30% and less than top 30% (grade 3) within the group, a user with a sleep score greater than or equal to P7 and less than P8 may be greater than or equal to the top 30% and less than top 10% (grade 2) within the group, and a user with a sleep score greater than or equal to P8 may be greater than or equal to the top 10% (grade 1) in the group. If the sleep score of the user of the electronic device 101 is 60 (point 822), the processor 350 may determine the sleep grade of the user of the electronic device 101 as grade 2.

In the above example, according to the sleep score distribution, the sleep grades are exemplified as including a total of five grades, but is the sleep grades are not limited thereto. For example, according to the sleep score distribution, the sleep grades may include a number of grades greater than or equal to five.

In the above example, the processor 350 may determine, based on the user's sleep score, the user information, and the sleep score distribution of the group corresponding to the user information about the user of the electronic device, the user's sleep grade. In some examples, based on the sleep scores of the user, the user information, and the distribution **of** sleep scores of the groups corresponding to the user information about the user of the electronic device 101, the processor 350 may determine a rank of the sleep score of the user within the group (e.g., 1500 out of 10000), or a percentage from the top (or bottom) within the group (e.g., the top 25% within the group).

In operation 709, in an embodiment, the processor 350 may display information related to the user's sleep grade through the display 320. For example, the processor 350 may display, through the display 320, information indicating that the user's sleep score corresponds to a third grade among a total of five sleep grades in the group. However, the information related to the sleep grade displayed through the display 320 is not limited to the above example.

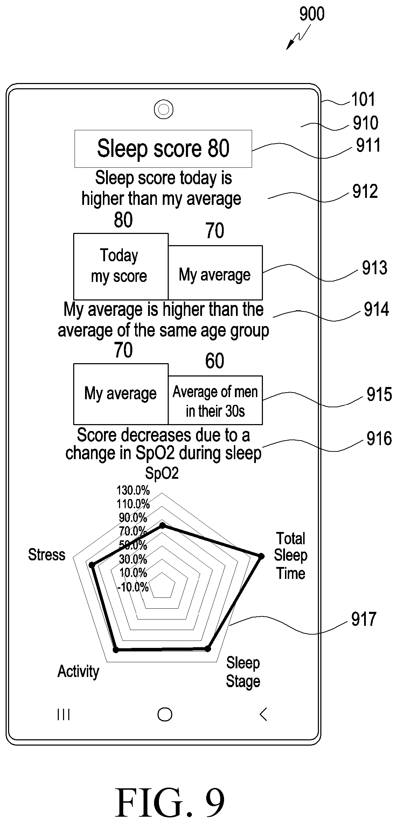

FIG. 9 is a diagram 900 illustrating a method of providing information about sleep quality according to various embodiments.

Referring to FIG. 9, in an embodiment, the processor 350 may display, through the display 320, information about at least one of the determined sleep score (e.g., currently (today) determined sleep score)), the average of the sleep scores determined during the designated period, average sleep score of the group having user information the same as or similar to the user information about the electronic device 101, and the value of the sleep factor related to a decrease (or increase) of the sleep score.

For example, as shown in FIG. 9, the processor 350 may display, through the screen 910, information 911 indicating the determined sleep score, information 912, 913 indicating that the determined sleep score is higher or lower than the sleep score obtained during a designated period (e.g., one month or one week), information 914, 915 for comparing the determined sleep score and the average of the sleep score of the group corresponding to the user information of the user of the electronic device 101, and information 917 indicating the value of the sleep factor related to the decrease in sleep score. However, the information about the sleep quality displayed by the processor 350 through the display 320 is not limited to the above example.

A method for providing information about sleep quality in an electronic device 101 according to various embodiments of the present disclosure may include obtaining, from an external electronic device through a communication circuitry (e.g., communication module 310) of the electronic device 101, sleep-related information including at least one of an exercise amount, a stress, or a saturation pulse oxygen of a user, based on the sleep-related information, determining the sleep quality of the user, and displaying information about the determined sleep quality of the user through a display 320 of the electronic device 101.

In various embodiments, the sleep-related information may further include at least one of a time of a third sleep stage in a first sleep cycle, a ratio of the third sleep stage to a total sleep time, a number of sleep cycles in the total sleep time, the total sleep time, a time of a first sleep cycle, an awake time in a first sleep stage, a time of a WASO in the first sleep stage, a ratio of the time of the WASO to the total sleep time, an awake time in the first sleep cycle, and a ratio of the awake time to the total sleep time.

In various embodiments, determining the sleep quality of the user may include calculating a sleep score of the user, by performing scoring on each of information included in the sleep-related information.

In various embodiments, calculating the sleep score of the user may include adding a value to a score corresponding to an amount of exercise obtained before a designated time and subtracting a value from a score corresponding to an amount of exercise obtained after the designated time.

In various embodiments, calculating the sleep score of the user may include assigning a lower score as the stress increases.

In various embodiments, calculating the sleep score of the user may include assigning a lower score based on a number of times being higher, the number of times being a number of times by which the stress of the user decreases being greater than or equal to a designated value within a designated time during sleep.

In various embodiments, determining the sleep quality of the user may further include, based on an average and standard deviation of sleep scores obtained during a designated period of time (i.e., the obtained information about the sleep quality of the user may include the average and standard deviation that are obtained during the designated period of time), calculating a designated range related to a sleep score, and the method may further include, correcting the calculated sleep score to the lower limit value if the calculated sleep score is less than a lower limit value of the designated range, and correcting the calculated sleep score to the upper limit value if the calculated sleep score is less than an upper limit value of the designated range.

In various embodiments, determining the sleep quality of the user may include based on a user input, obtaining information about an amount of beverage intake of a user, and based on the amount of the beverage intake, the sleep-related information, the information about the sleep quality, and the user information, determining the sleep quality of the user.

In various embodiments, determining the sleep quality of the user may include obtaining a sleep score distribution of a group corresponding to the user information, and based on the user information and the sleep score distribution of the group, determining a sleep grade of the user in the group.

In various embodiments, the user information may include a gender, an age, and/or a height/weight of the user.

According to various embodiments of the present disclosure, a method for providing information about sleep quality and an electronic device supporting the same may more accurately evaluate sleep quality, based on various information related to sleep and obtainable through a wearable device and/or a user input.

In addition, based on information about a user of the electronic device, comparison information between a sleep score of the user of the electronic device and the sleep scores of other users having user information identical or similar to the information about the user of the electronic device may be provided.

In addition, the structure of data used in the above-described embodiments of the present disclosure may be recorded on a computer-readable recording medium through various means. The computer-readable recording medium includes storage media such as magnetic storage media (e.g., ROM, floppy disk, hard disk, etc.) and optical reading media (e.g., CD-ROM, DVD, etc.).

The embodiments of the disclosure disclosed in the specification and the drawings provide merely specific examples to easily describe technical content according to the embodiments of the disclosure and aid understanding of the embodiments of the disclosure, not intended to limit the scope of the embodiments of the disclosure.

## Claims

1. An electronic device (101) comprising:
a sensor (176) including an acceleration sensor and a photoplethysmogram, PPG, sensor;
a display (320);
at least one processor (350) operatively coupled with the sensor (176) and the display (320); and
memory (340) storing instructions that, when executed by the at least one processor (350), cause the electronic device to:
obtain (401), using the sensor (176), first information during a day and second information during a sleep, the first information including an exercise amount of a user during a non-sleep period,
based on both the first information and the second information, determine (403) a sleep quality score of the user, and
display (405) information about the determined sleep quality score of the user through the display (320),
wherein the memory (340) stores scores corresponding to designated ranges associated with the first information and the second information,
wherein based on the scores and the designated ranges, a first score corresponding to a first designated range to which the first information belongs and a second score corresponding to a second designated range to which the second information belongs are obtained, and
wherein based on the first score and the second score, the sleep quality score of the user is determined.

2. The electronic device of claim 1, wherein the first information further includes a stress of the user during the non-sleep period, and
wherein the second information includes at least one of a saturation pulse oxygen during the sleep, a sleep time during the sleep, or a sleep stage of the user during the sleep.

3. The electronic device of claim 2, wherein the first score is determined based on a score corresponding to a designated range to which the exercise amount belongs among the designated ranges and a score corresponding to a designated range to which the stress belongs the designated ranges, and
wherein the second score is determined based on a score corresponding to a designated range to which the at least one of the saturation pulse oxygen, the sleep time during the sleep, or the sleep stage belongs among the designated ranges.

4. The electronic device of claim 2, wherein the instructions that, when executed by the at least one processor (350), further cause the electronic device (101) to:
assign a higher score as an amount of exercise obtained before a designated time increases; and
assign a lower score as an amount of exercise obtained after the designated time increases.

5. The electronic device of claim 3, wherein the instructions that, when executed by the at least one processor (350), further cause the electronic device (101) to:
assign a lower score as the stress of the user increases, and
assign a lower score, based on a number of times being higher, the number of times being a number of times by which the saturation pulse oxygen of the user decreases being greater than or equal to a designated value within a designated time during sleep.

6. The electronic device of claim 3, wherein the instructions that, when executed by the at least one processor (350), further cause the electronic device (101) to:
determine a designated range related to the determined sleep quality score based on an average of sleep quality scores and a standard deviation of the sleep quality scores obtained during the designated period of time,
based on the determined sleep quality score being less than a lower limit value of the designated range, correct the determined sleep quality score to the lower limit value, and
based on the determined sleep quality score being less than an upper limit value of the designated range, correct the determined sleep quality score to the upper limit value.

7. The electronic device of claim 3, wherein the instructions that, when executed by the at least one processor (350), further cause the electronic device (101) to:
based on a user input, obtain information about an amount of beverage intake of the user, and
wherein the sleep quality score of the user is determined based on the amount of the beverage intake of the user, the first information, and the first information.

8. The electronic device of claim 1, wherein the instructions that, when executed by the at least one processor (350), further cause the electronic device (101) to:
obtain user information of the user,
obtain a sleep quality score distribution of a group corresponding to the user information, and
based on the user information and the sleep quality score distribution of the group, determine a sleep grade of the user.

9. A computer implemented method for providing information about sleep quality, the method comprising
obtaining (401), using a sensor (176) of an electronic device (101), first information during a day and second information during a sleep, the first information including an exercise amount of a user during a non-sleep period, the sensor (176) including an acceleration sensor and a photoplethysmogram, PPG, sensor;
based on both the first information and the second information, determining (403) a sleep quality score of the user; and
displaying (405) information about the determined sleep quality score of the user through a display (320) of the electronic device (101),
wherein memory (340) of electronic device (101) stores scores corresponding to designated ranges associated with the first information and the second information,
wherein based on the scores and the designated ranges, a first score corresponding to a first designated range to which the first information belongs and a second score corresponding to a second designated range to which the second information belongs are obtained, and
wherein based on the first score and the second score, the sleep quality score of the user is determined.

10. The method of claim 9, wherein the first information further includes a stress of the user during the non-sleep period, and
wherein the second information includes at least one of a saturation pulse oxygen during the sleep, a sleep time during the sleep, or a sleep stage of the user during the sleep.

11. The method of claim 9, wherein determining the sleep quality score of the user comprises:
assign a higher score as an amount of exercise obtained before a designated time increases; and
assign a lower score as an amount of exercise obtained after the designated time increases.

12. The method of claim 9, wherein determining the sleep quality score of the user comprises:
assigning a lower score as a stress of the user increases, and
assigning a lower score based on a number of times by which a saturation pulse oxygen of the user decreases being greater than or equal to a designated value within a designated time during sleep.

13. The method of claim 9,
wherein determining the sleep quality score of the user further comprises:
determining a designated range related to the determined sleep quality score based on an average of sleep quality scores and a standard deviation of the sleep quality scores obtained during a designated period of time; and
based on the determined sleep quality score being less than a lower limit value of the designated range, correcting the determined sleep quality score to the lower limit value; and
based on the determined sleep quality score being less than an upper limit value of the designated range, correcting the determined sleep quality score to the upper limit value.

14. The method of claim 9, wherein determining the sleep quality score of the user comprises:
obtaining a sleep quality score distribution of a group corresponding to the user information; and
based on the user information and the sleep quality score distribution of the group, determining a sleep grade of the user.

## Patentansprüche

1. Elektronische Vorrichtung (101), umfassend:
einen Sensor (176), der einen Beschleunigungssensor und einen Photoplethysmogrammsensor, PPG-Sensor, enthält;
eine Anzeige (320);
mindestens einen Prozessor (350), der mit dem Sensor (176) und der Anzeige (320) wirkgekoppelt ist; und
einen Speicher (340), der Anweisungen speichert, die, wenn sie durch den mindestens einen Prozessor (350) ausgeführt werden, die elektronische Vorrichtung zu Folgendem veranlassen:
Erlangen (401), unter Verwendung des Sensors (176), erster Informationen während eines Tages und zweiter Informationen während eines Schlafes, wobei die ersten Informationen eine Trainingsmenge eines Benutzers während einer Nicht-Schlaf-Periode enthalten,
basierend auf sowohl den ersten Informationen als auch den zweiten Informationen, Bestimmen (403) einer Schlafqualitätsbewertung des Benutzers und
Anzeigen (405) von Informationen über die bestimmte Schlafqualitätsbewertung des Benutzers durch die Anzeige (320),
wobei der Speicher (340) Bewertungen speichert, die festgelegten Bereichen entsprechen, die den ersten Informationen und den zweiten Informationen zugeordnet sind,
wobei basierend auf den Bewertungen und den festgelegten Bereichen eine erste Bewertung, die einem ersten festgelegten Bereich entspricht, zu dem die ersten Informationen gehören, und eine zweite Bewertung, die einem zweiten festgelegten Bereich entspricht, zu dem die zweiten Informationen gehören, erlangt werden, und
wobei basierend auf der ersten Bewertung und der zweiten Bewertung die Schlafqualitätsbewertung des Benutzers bestimmt wird.

2. Elektronische Vorrichtung nach Anspruch 1, wobei die ersten Informationen ferner einen Stress des Benutzers während der Nicht-Schlaf-Periode enthalten und
wobei die zweiten Informationen mindestens eines von einer Puls-Sauerstoffsättigung während des Schlafes, einer Schlafzeit während des Schlafes oder einer Schlafphase des Benutzers während des Schlafes enthalten.

3. Elektronische Vorrichtung nach Anspruch 2, wobei die erste Bewertung basierend auf einer Bewertung, die einem festgelegten Bereich entspricht, zu dem die Trainingsmenge unter den festgelegten Bereichen gehört, und einer Bewertung, die einem festgelegten Bereich entspricht, zu dem der Stress unter den festgelegten Bereichen gehört, bestimmt wird und
wobei die zweite Bewertung basierend auf einer Bewertung bestimmt wird, die einem festgelegten Bereich entspricht, zu dem das mindestens eine von der Puls-Sauerstoffsättigung, der Schlafzeit während des Schlafens oder der Schlafphase unter den festgelegten Bereichen gehört.

4. Elektronische Vorrichtung nach Anspruch 2, wobei die Anweisungen, die, wenn sie durch den mindestens einen Prozessor (350) ausgeführt werden, die elektronische Vorrichtung (101) ferner zu Folgendem veranlassen:
Zuweisen einer höheren Bewertung, wenn sich eine Menge an Training, die vor einer festgelegten Zeit erlangt wird, erhöht; und
Zuweisen einer niedrigeren Bewertung, wenn sich eine Menge an Training, die nach der festgelegten Zeit erlangt wird, erhöht.

5. Elektronische Vorrichtung nach Anspruch 3, wobei die Anweisungen, die, wenn sie durch den mindestens einen Prozessor (350) ausgeführt werden, die elektronische Vorrichtung (101) ferner zu Folgendem veranlassen:
Zuweisen einer niedrigeren Bewertung, wenn sich der Stress des Benutzers erhöht, und
Zuweisen einer niedrigeren Bewertung basierend darauf, dass eine Anzahl von Malen höher ist, wobei die Anzahl von Malen eine Anzahl von Malen ist, um die die Puls-Sauerstoffsättigung des Benutzers abnimmt und die größer als oder gleich einem festgelegten Wert innerhalb einer festgelegten Zeit während des Schlafens ist.

6. Elektronische Vorrichtung nach Anspruch 3, wobei die Anweisungen, die, wenn sie durch den mindestens einen Prozessor (350) ausgeführt werden, die elektronische Vorrichtung (101) ferner zu Folgendem veranlassen:
Bestimmen eines festgelegten Bereichs in Bezug auf die bestimmte Schlafqualitätsbewertung basierend auf einem Durchschnitt von Schlafqualitätsbewertungen und einer Standardabweichung der Schlafqualitätsbewertungen, die während des festgelegten Zeitraums erlangt werden,
basierend darauf, dass die bestimmte Schlafqualitätsbewertung geringer als ein unterer Grenzwert des festgelegten Bereichs ist, Korrigieren der bestimmten Schlafqualitätsbewertung auf den unteren Grenzwert und
basierend darauf, dass die bestimmte Schlafqualitätsbewertung geringer als ein oberer Grenzwert des festgelegten Bereichs ist, Korrigieren der bestimmten Schlafqualitätsbewertung auf den oberen Grenzwert.

7. Elektronische Vorrichtung nach Anspruch 3, wobei die Anweisungen, die, wenn sie durch den mindestens einen Prozessor (350) ausgeführt werden, die elektronische Vorrichtung (101) ferner zu Folgendem veranlassen:
basierend auf einer Benutzereingabe, Erlangen von Informationen über eine Getränkeaufnahmemenge des Benutzers, und
wobei die Schlafqualitätsbewertung des Benutzers basierend auf der Menge der Getränkeaufnahme des Benutzers, den ersten Informationen und den ersten Informationen bestimmt wird.

8. Elektronische Vorrichtung nach Anspruch 1, wobei die Anweisungen, die, wenn sie durch den mindestens einen Prozessor (350) ausgeführt werden, die elektronische Vorrichtung (101) ferner zu Folgendem veranlassen:
Erlangen von Benutzerinformationen des Benutzers,
Erlangen einer Schlafqualitätsbewertungsverteilung einer Gruppe, die den Benutzerinformationen entspricht, und,
basierend auf den Benutzerinformationen und der Schlafqualitätsbewertungsverteilung der Gruppe, Bestimmen eines Schlafgrades des Benutzers.

9. Computerimplementiertes Verfahren zum Bereitstellen von Informationen über Schlafqualität, wobei das Verfahren Folgendes umfasst:
Erlangen (401), unter Verwendung eines Sensors (176) einer elektronischen Vorrichtung (101), erster Informationen während eines Tages und zweiter Informationen während eines Schlafes, wobei die ersten Informationen eine Trainingsmenge eines Benutzers während einer Nicht-Schlaf-Periode enthalten, wobei der Sensor (176) einen Beschleunigungssensor und einen Photoplethysmogrammsensor, PPG-Sensor, enthält;
basierend auf sowohl den ersten Informationen als auch den zweiten Informationen, Bestimmen (403) einer Schlafqualitätsbewertung des Benutzers; und
Anzeigen (405) von Informationen über die bestimmte Schlafqualitätsbewertung des Benutzers durch eine Anzeige (320) der elektronischen Vorrichtung (101),
wobei Speicher (340) der elektronischen Vorrichtung (101) Bewertungen speichert, die festgelegten Bereichen entsprechen, die den ersten Informationen und den zweiten Informationen zugeordnet sind,
wobei basierend auf den Bewertungen und den festgelegten Bereichen eine erste Bewertung, die einem ersten festgelegten Bereich entspricht, zu dem die ersten Informationen gehören, und eine zweite Bewertung, die einem zweiten festgelegten Bereich entspricht, zu dem die zweiten Informationen gehören, erlangt werden, und
wobei basierend auf der ersten Bewertung und der zweiten Bewertung die Schlafqualitätsbewertung des Benutzers bestimmt wird.

10. Verfahren nach Anspruch 9, wobei die ersten Informationen ferner einen Stress des Benutzers während der Nicht-Schlaf-Periode enthalten und
wobei die zweiten Informationen mindestens eines von einer Puls-Sauerstoffsättigung während des Schlafes, einer Schlafzeit während des Schlafes oder einer Schlafphase des Benutzers während des Schlafes enthalten.

11. Verfahren nach Anspruch 9, wobei das Bestimmen der Schlafqualitätsbewertung des Benutzers Folgendes umfasst:
Zuweisen einer höheren Bewertung, wenn sich eine Menge an Training, die vor einer festgelegten Zeit erlangt wird, erhöht; und
Zuweisen einer niedrigeren Bewertung, wenn sich die Menge an Training, die nach der festgelegten Zeit erlangt wird, erhöht.

12. Verfahren nach Anspruch 9, wobei das Bestimmen der Schlafqualitätsbewertung des Benutzers Folgendes umfasst:
Zuweisen einer niedrigeren Bewertung, wenn sich ein Stress des Benutzers erhöht, und
Zuweisen einer niedrigeren Bewertung basierend darauf, dass eine Anzahl von Malen, um die eine Puls-Sauerstoffsättigung des Benutzers abnimmt, größer als oder gleich einem festgelegten Wert innerhalb einer festgelegten Zeit während des Schlafens ist.

13. Verfahren nach Anspruch 9,
wobei das Bestimmen der Schlafqualitätsbewertung des Benutzers ferner Folgendes umfasst:
Bestimmen eines festgelegten Bereichs in Bezug auf die bestimmte Schlafqualitätsbewertung basierend auf einem Durchschnitt von Schlafqualitätsbewertungen und einer Standardabweichung der Schlafqualitätsbewertungen, die während eines festgelegten Zeitraums erlangt werden; und
basierend darauf, dass die bestimmte Schlafqualitätsbewertung geringer als ein unterer Grenzwert des festgelegten Bereichs ist, Korrigieren der bestimmte Schlafqualitätsbewertung auf den unteren Grenzwert; und
basierend darauf, dass die bestimmte Schlafqualitätsbewertung geringer als ein oberer Grenzwert des festgelegten Bereichs ist, Korrigieren der bestimmten Schlafqualitätsbewertung auf den oberen Grenzwert.

14. Verfahren nach Anspruch 9, wobei das Bestimmen der Schlafqualitätsbewertung des Benutzers Folgendes umfasst:
Erlangen einer Schlafqualitätsbewertungsverteilung einer Gruppe, die den Benutzerinformationen entspricht; und,
basierend auf den Benutzerinformationen und der Schlafqualitätsbewertungsverteilung der Gruppe, Bestimmen eines Schlafgrades des Benutzers.

## Revendications

1. Dispositif électronique (101) comprenant :
un capteur (176) comprenant un capteur d'accélération et un capteur de photopléthysmogramme, PPG ;
un affichage (320) ;
au moins un processeur (350) couplé fonctionnellement au capteur (176) et à l'affichage (320) ; et
de la mémoire (340) stockant des instructions qui, lorsqu'elles sont exécutées par l'au moins un processeur (350), amènent le dispositif électronique à :
obtenir (401), à l'aide du capteur (176), de premières informations pendant une journée et de secondes informations pendant un sommeil, les premières informations comprenant une quantité d'exercice d'un utilisateur pendant une période de non sommeil,
sur la base des premières informations et des secondes informations, déterminer (403) un score de qualité du sommeil de l'utilisateur, et
afficher (405) des informations concernant le score de qualité de sommeil déterminé de l'utilisateur au travers de l'affichage (320),
dans lequel la mémoire (340) stocke des scores correspondant à des plages désignées associées aux premières informations et aux secondes informations,
dans lequel, sur la base des scores et des plages désignées, un premier score correspondant à une première plage désignée à laquelle appartiennent les premières informations et un second score correspondant à une seconde plage désignée à laquelle appartiennent les secondes informations sont obtenus, et
dans lequel, sur la base du premier score et du second score, le score de qualité du sommeil de l'utilisateur est déterminé.

2. Dispositif électronique de la revendication 1, dans lequel les premières informations comprennent en outre un stress de l'utilisateur pendant la période de non sommeil, et
dans lequel les secondes informations comprennent au moins un élément parmi une saturation pulsée en oxygène pendant le sommeil, un temps de sommeil pendant le sommeil, ou un stade de sommeil de l'utilisateur pendant le sommeil.

3. Dispositif électronique de la revendication 2, dans lequel le premier score est déterminé sur la base d'un score correspondant à une plage désignée à laquelle appartient la quantité d'exercice parmi les plages désignées et d'un score correspondant à une plage désignée à laquelle appartient le stress parmi les plages désignées, et
dans lequel le second score est déterminé sur la base d'un score correspondant à une plage désignée à laquelle appartient l'au moins un élément parmi la saturation pulsée en oxygène, le temps de sommeil pendant le sommeil ou le stade de sommeil parmi les plages désignées.

4. Dispositif électronique de la revendication 2, dans lequel les instructions qui, lorsqu'elles sont exécutées par l'au moins un processeur (350), amènent en outre le dispositif électronique (101) à :
attribuer un score plus élevé lorsque la quantité d'exercice obtenue augmente avant un temps désigné ; et
attribuer un score plus bas lorsque la quantité d'exercice obtenue augmente après un temps désigné.

5. Dispositif électronique de la revendication 3, dans lequel les instructions qui, lorsqu'elles sont exécutées par l'au moins un processeur (350), amènent en outre le dispositif électronique (101) à :
attribuer un score plus bas lorsque le stress de l'utilisateur augmente, et
attribuer un score plus bas, sur la base du fait qu'un nombre de fois est supérieur, le nombre de fois étant un nombre de fois par lequel la saturation pulsée en oxygène de l'utilisateur diminue étant supérieur ou égal à une valeur désignée dans les limites d'un temps désigné pendant le sommeil.

6. Dispositif électronique de la revendication 3, dans lequel les instructions qui, lorsqu'elles sont exécutées par l'au moins un processeur (350), amènent en outre le dispositif électronique (101) à :
déterminer une plage désignée liée au score de qualité du sommeil déterminé sur la base d'une moyenne des scores de qualité du sommeil et d'un écart type des scores de qualité du sommeil obtenus pendant la période temporelle désignée,
sur la base du fait que le score de qualité de sommeil déterminé est inférieur à une valeur de limite basse de la plage désignée, corriger le score de qualité de sommeil déterminé à la valeur de limite basse, et
sur la base du fait que le score de qualité de sommeil déterminé est inférieur à une valeur de limite haute de la plage désignée, corriger le score de qualité de sommeil déterminé à la valeur de limite haute.

7. Dispositif électronique de la revendication 3, dans lequel les instructions qui, lorsqu'elles sont exécutées par l'au moins un processeur (350), amènent en outre le dispositif électronique (101) à :
sur la base d'une saisie de l'utilisateur, obtenir des informations concernant une quantité de boisson consommée par l'utilisateur, et
dans lequel le score de qualité du sommeil de l'utilisateur est déterminé sur la base de la quantité de boisson consommée par l'utilisateur, des premières informations et des premières informations.

8. Dispositif électronique de la revendication 1, dans lequel les instructions qui, lorsqu'elles sont exécutées par l'au moins un processeur (350), amènent en outre le dispositif électronique (101) à :
obtenir des informations d'utilisateur de l'utilisateur,
obtenir une distribution des scores de qualité du sommeil d'un groupe correspondant aux informations d'utilisateur, et
sur la base des informations d'utilisateur et de la distribution du score de qualité du sommeil du groupe, déterminer un niveau de sommeil de l'utilisateur.

9. Procédé mis en œuvre par ordinateur pour fournir des informations concernant la qualité du sommeil, le procédé comprenant
l'obtention (401), à l'aide d'un capteur (176) d'un dispositif électronique (101), de premières informations pendant une journée et de secondes informations pendant un sommeil, les premières informations comprenant une quantité d'exercice d'un utilisateur pendant une période de non sommeil, le capteur (176) comprenant un capteur d'accélération et un capteur de photopléthysmogramme, PPG ;
sur la base des premières informations et des secondes informations, la détermination (403) d'un score de qualité du sommeil de l'utilisateur, et
l'affichage (405) d'informations concernant le score de qualité de sommeil déterminé de l'utilisateur à travers un affichage (320) du dispositif électronique (101),
dans lequel la mémoire (340) du dispositif électronique (101) stocke des scores correspondant à des plages désignées associées aux premières informations et aux secondes informations,
dans lequel, sur la base des scores et des plages désignées, un premier score correspondant à une première plage désignée à laquelle appartiennent les premières informations et un second score correspondant à une seconde plage désignée à laquelle appartiennent les secondes informations sont obtenus, et
dans lequel, sur la base du premier score et du second score, le score de qualité du sommeil de l'utilisateur est déterminé.

10. Procédé de la revendication 9, dans lequel les premières informations comprennent en outre un stress de l'utilisateur pendant la période de non sommeil, et
dans lequel les secondes informations comprennent au moins un élément parmi une saturation pulsée en oxygène pendant le sommeil, un temps de sommeil pendant le sommeil, ou un stade de sommeil de l'utilisateur pendant le sommeil.

11. Procédé de la revendication 9, dans lequel la détermination du score de qualité de sommeil de l'utilisateur comprend :
attribuer un score plus élevé lorsque la quantité d'exercice obtenue augmente avant un temps désigné ; et
attribuer un score plus bas lorsque la quantité d'exercice obtenue augmente après un temps désigné.

12. Procédé de la revendication 9, dans lequel la détermination du score de qualité de sommeil de l'utilisateur comprend :
l'attribution d'un score plus bas lorsque le stress de l'utilisateur augmente, et
l'attribution d'un score plus bas sur la base du fait qu'un nombre de fois par lequel une saturation pulsée en oxygène de l'utilisateur diminue est supérieur ou égal à une valeur désignée dans les limites d'un temps désigné pendant le sommeil.

13. Procédé de la revendication 9,
dans lequel la détermination du score de qualité de sommeil de l'utilisateur comprend en outre :
la détermination d'une plage désignée liée au score de qualité du sommeil déterminé sur la base d'une moyenne des scores de qualité du sommeil et d'un écart type des scores de qualité du sommeil obtenus pendant une période temporelle désignée ; et
sur la base du fait que le score de qualité de sommeil déterminé est inférieur à une valeur de limite basse de la plage désignée, la correction du score de qualité de sommeil déterminé à la valeur de limite basse ; et
sur la base du fait que le score de qualité de sommeil déterminé est inférieur à une valeur de limite haute de la plage désignée, la correction du score de qualité de sommeil déterminé à la valeur de limite haute.

14. Procédé de la revendication 9, dans lequel la détermination du score de qualité de sommeil de l'utilisateur comprend :
l'obtention d'une distribution des scores de qualité du sommeil d'un groupe correspondant aux informations d'utilisateur ; et
sur la base des informations d'utilisateur et de la distribution du score de qualité du sommeil du groupe, la détermination d'un niveau de sommeil de l'utilisateur.
